(19) Europäisches Patentamt
European Patent Office
Office européen des brevets

(11) Veröffentlichungsnummer: **0 159 640**
**A1**

(12) # EUROPÄISCHE PATENTANMELDUNG

(21) Anmeldenummer: **85104503.9**

(22) Anmeldetag: **13.04.85**

(51) Int. Cl.⁴: **C 07 D 213/68**, C 07 D 413/06, A 61 K 31/44

(30) Priorität: **19.04.84 DE 3414830**

(43) Veröffentlichungstag der Anmeldung: **30.10.85 Patentblatt 85/44**

(84) Benannte Vertragsstaaten: **DE FR GB**

(71) Anmelder: **HOECHST AKTIENGESELLSCHAFT, Postfach 80 03 20, D-6230 Frankfurt am Main 80 (DE)**

(72) Erfinder: **Alreja, Bipin Dhirajlal, Dr., "The Beacon" 140 Backbay Reclamation, Bombay 400 021 (IN)**
Erfinder: **Kattige, Samba Laxminarayan, 14-132, Asha Kiran Garodia Nagar, Ghatkopar (East) Bombay 400 077 (IN)**
Erfinder: **de Souza, Noel John, Dr., 702 Avanti, Central Avenue, Santa Cruz (West) Bombay 400 054 (IN)**

(54) **4-Methoxy-2,2'-bipyridyl-6-aldoxime, Verfahren und Zwischenprodukte zu ihrer Herstellung und ihre Verwendung als Arzneimittel.**

(57) Die vorliegende Erfindung betrifft die Verbindung 4-Methoxy-2,2'-bipyridyl-6-aldoxim und Isomere und ein Verfahren sowie Zwischenprodukte zu dessen Herstellung. Die Verbindungen können bei der Behandlung von Krankheiten, die durch Bakterien oder Amöben hervorgerufen werden, verwendet werden.

EP 0 159 640 A1

# 4-Methoxy-2,2'-bipyridyl-6-aldoxime, Verfahren und Zwischenprodukte zu ihrer Herstellung und ihre Verwendung als Arzneimittel

Die vorliegende Erfindung betrifft ein neues Verfahren zur Herstellung von (E)-4-Methoxy-2,2'-bipyridyl-6-aldoxim der Formel I und seinem (Z)-Isomeren der Formel II:

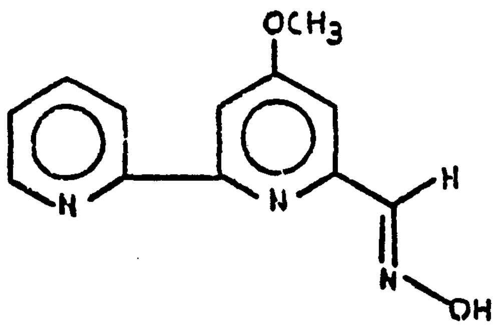

I

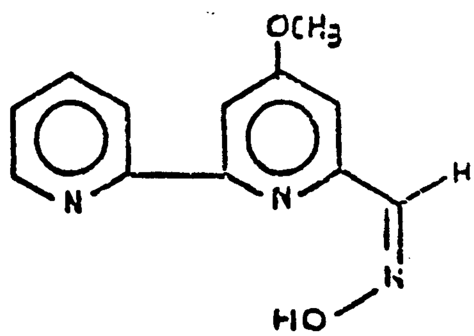

II

Die Erfindung betrifft ferner die Zwischenprodukte der Formeln IV, V und VI

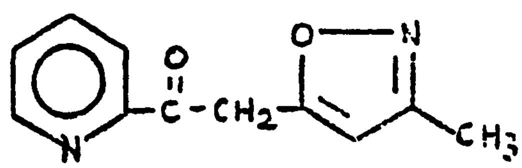

IV

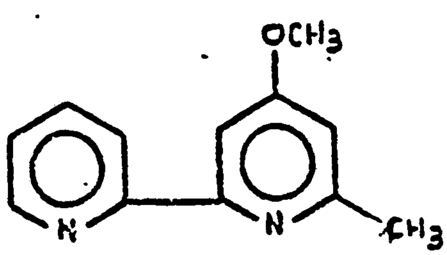

VI

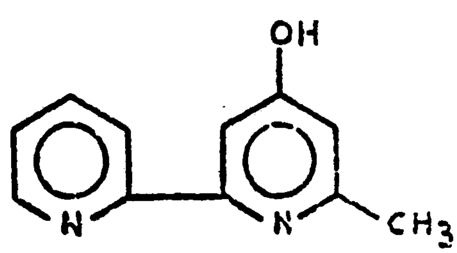

V

sowie ihre Herstellung.

Die Verbindung der Formel I ist als Caerulomycin bereits beschrieben worden (A. Funk und P.V. Divekar, Canadian

Journal of Microbiology, 5 (1959), 317). Sie wurde gewonnen aus einer Kultur des Mikroorganismus Streptomyces caeruleus und zeigt starke fungizide und herbizide Wirkungen. Die Struktur der Verbindung wurde aufgeklärt von P.V. Divekar et al. (Canadian Journal of Chemistry 45 (1967), 1215). Eine Synthese dieser Verbindung, ausgehend von 4-Methoxy-2,2'-bipyridyl-1-oxid, wurde berichtet von S. Ranganathan et al. (Canadian Journal of Chemistry 47 (1969) 165).

Das erfindungsgemäße Verfahren zur Herstellung der Verbindungen der Formeln I und II ist dadurch gekennzeichnet, daß man die Verbindung 3,5-Dimethylisoxazol der Formel III

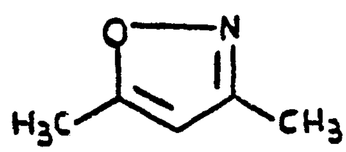

III

mit Lithiumdiisopropylamid umsetzt zu der Verbindung (3'-Methyl-5'-isoxazolyl)-2-acetylpyridin der Formel IV

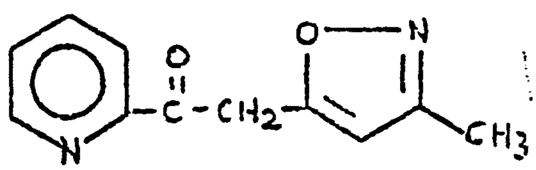

IV

die erhaltene Verbindung hydriert zu der Verbindung 4-Hydroxy-6-methyl-2,2'-bipyridyl der Formel V

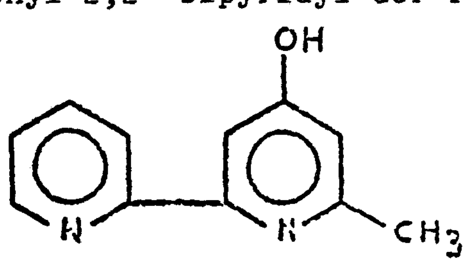

V

die erhaltene Verbindung mit Diazomethan zu 4-Methoxy-6-methyl-2,2'-bipyridyl der Formel VI

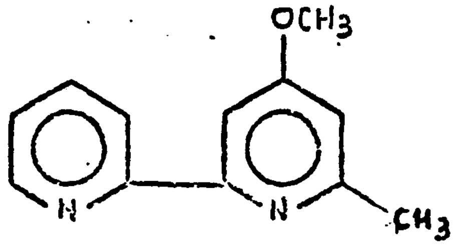

VI

umsetzt, die erhaltene Verbindung wiederum umsetzt mit Lithiumdiisopropylamid, wobei man eine Mischung aus den Verbindungen der Formel I und II erhält, und diese auf chromatographischem Wege in die einzelnen Isomeren trennt.

Die Umsetzung der Verbindung der Formel III mit Lithiumdiisopropylamid wird in wasserfreien Äthern, wie z.B. Tetrahydrofuran oder Äthylpyridin-2-carboxylat bei einer Temperatur unterhalb von -60°C durchgeführt.
Die Hydrierung der Verbindung der Formel IV erfolgt in Gegenwart eines üblichen Katalysators, vorzugsweise von Platinoxid. Die Verbindung der Formel V wird aus der Reaktionsmischung in üblicher Weise isoliert, z.B. durch Abfiltrieren des verbrauchten Katalysators, Behandeln der Lösung mit Essigsäure, gefolgt durch Eindampfen im Vakuum und anschließender Kristallisation.
Die Umsetzung der Verbindung der Formel V mit Diazomethan erfolgt auf übliche Weise, ebenfalls die Isolation der erhaltenen Verbindung der Formel VI aus der Reaktionsmischung. Zum Beispiel wird die Reaktionsmischung unter Stickstoff konzentriert und dann über Aluminiumoxid chromatographiert.
Die Verbindung der Formel VI wird mit Lithiumdiisopropylamid in einem wasserfreien Äther, vorzugsweise in Tetrahydrofuran oder Butylnitrit, bei einer Temperatur unterhalb

von -60°C umgesetzt. Dabei erhält man eine Mischung des (E)- und (Z)-Isomere der Formeln I und II.

Die Trennung der Isomere wird ebenfalls in üblicher Weise durchgeführt, z.B. durch Chromatographie an Aluminiumoxid gefolgt durch Chromatographie an Silikagel.

Das (Z)-Isomer der Formel II ist noch nicht beschrieben worden und damit neu.

Die Zwischenverbindungen der Formeln IV, V und VI sind ebenfalls neu.

Zusätzlich zu den bereits bekannten pharmakologischen Wirkungen von Caerulomycin der Formel I wurde gefunden, daß diese Verbindung ebenfalls coccidiostatische Eigenschaften hat und gegen Amöben wirksam ist. Das (Z)-Isomere der Formel II besitzt antibakterielle Eigenschaften und ist ebenfalls wirksam gegen Amöben.

Beispiel 1

(3'-Methyl-5'-isoxazolyl)-2-acetylpyridin (IV)

Zu einer frisch hergestellten Lösung von Lithiumdiisopropylamid [aus Diisopropylamin (35.7 g) und n-Butyllithium in Hexan (234 ml einer 1,55 M-Lösung)], die unter Rühren auf -70°C abgekühlt wurde, tropft man in einer inerten Atmosphäre eine Lösung aus 3,5-Dimethylisoxazol (32 g) in wasserfreiem Tetrahydrofuran (100 ml). Nach beendeter Zugabe rührt man noch 1 Stunde bei -70°C und gibt dann eine Pyridin-2-ethylcarboxylatlösung (50 g in 300 ml Tetrahydrofuran) zu, wobei man die Temperatur bei -70°C hält. Dann rührt man das Reaktionsgemisch 1 Stunde lang und läßt es mit einer Ammoniumchloridlösung (100 ml) reagieren. Die organische Schicht trennt man ab, und die wäßrige Lösung wird dreimal mit je 300 ml Ethylacetat extrahiert. Die Extrakte werden mit der organischen Schicht vereinigt, mit Wasser gewaschen, über wasserfreiem Natriumsulfat getrocknet und zu einem Rückstand eingeengt, der chromatographisch über eine Kieselgelsäule gereinigt wird. Die gewünschte Verbindung erhält man in dem Laufmittelgemisch aus Petroleumäther und Ethylacetat (4:1) als Feststoff, der dann aus Diisopropyläther umkristallisiert wird. Ausbeute 58 g, Schmelzpunkt 250 - 254°C (Zers.).

Beispiel 2

4-Hydroxy-6-methyl-2,2'-bypyridyl (V)

Man hydriert eine Lösung von (3'-Methyl-5'-isoxazolyl)-2-acetylpyridin (16 g) in Methanol (300 ml) bei Zimmertemperatur unter normalem Druck in Gegenwart von Platinoxid (1,0 g) 1 1/2 Stunden. Dann filtriert man, um den Katalysator zu entfernen und engt im Vakuum bis auf ein Volumen von 100 ml ein. Nach Zugabe von Essigsäure zur konzentrierten Lösung rührt man das Gemisch und erhitzt es 2 Stunden auf 60 - 70°C. Nach Eindampfen des Reaktionsgemisches zur

Trockene und Umkristallisation des Rückstandes aus Methanol:Diisopropyläther erhält man die gewünschte Verbindung in einer Ausbeute von 92 %. Schmelzpunkt 170 - 172°C.

Beispiel 3

4-Methoxy-6-methyl-2,2'-bipyridyl (VI)

Man gibt zu einer Lösung aus 4-Hydroxy-6-methyl-2,2'-bipyridyl (12 g) in Methanol unter kräftigem Rühren eine Lösung aus Diazomethan (6,7) in Äther (200 ml) und kühlt auf 0°C ab. Dann beläßt man das Reaktionsgemisch 3 Stunden bei 0°C, engt in einer Stickstoffatmosphäre zu einem Rückstand ein, der chromatographisch über eine neutrale Aluminiumoxidsäule gereinigt wird. Unter Verwendung eines Gemisches aus Petroleumäther und Ethylacetat (95:5) als Laufmittel erhält man die gewünschte Verbindung in einer Ausbeute von 80 %. Schmelzpunkt 59 - 60°C.

Beispiel 4

(Z)-4-Methoxy-2,2'-bipyridyl-6-aldoxim (II)

Zu einer frisch hergestellten Lösung von Lithiumdiisopropylamid (0,2 Mol) in Hexan (150 ml) tropft man eine Lösung aus 4-Methoxy-6-methyl-2,2'-bipyridyl (20 g) in wasserfreiem THF (200 ml) und kühlt unter Rühren auf -70°C ab. Nach 45-minütigem Rühren gibt man Butylnitrit (23,2 g) bei -70°C zu und rührt nochmals 1 Stunde. Dann gibt man eine wäßrige Ammoniumsulfatlösung zu und läßt sich das Reaktionsgemisch auf Zimmertemperatur erwärmen. Die organische Schicht trennt man vom Reaktionsgemisch ab und extrahiert die wäßrige Schicht mit Ethylacetat. Die vereinigten organischen Schichten wäscht man mit Wasser, trocknet über Natriumsulfat und engt ein. Den erhaltenen Rückstand reinigt man zunächst chromatographisch über eine Aluminiumoxidsäule. Die endgültige Reinigung erfolgt über eine Kieselgelsäule unter Verwendung von Chloroform und anschließend einer Mischung von Chloroform mit 1 % Ammoniumhydroxidlösung. Beim Um-

kristallisieren aus Ethylacetat/Petroleumäther erhält man das (Z)-4-Methoxy-2,2'-bipyridyl-6-aldoxim (6,66 g) mit einem Schmelzpunkt von 172-174°C.

Beispiel 5

(E)-4-Methoxy-2,2'-bipyridyl-6-aldoxim (I)

Man verfährt wie in Beispiel 4, bis man nach Isolierung des reinen (Z)-4-Methoxy-2,2'-bipyridyl-6-aldoxims und Eluierung mit Chloroform (mit 1 % Ammoniumhydroxidlösung) das reine (E)-4-Methoxy-2,2'-bipyridyl-6-aldoxim (3,75 g) erhält, das aus Methanol umkristallisiert wird. Schmelzpunkt 172 - 174°C.

0159640
HOE 84/F 091

Patentansprüche:

1. (Z)-4-Methoxy-2,2'-bipyridyl-6-aldoxim der Formel II

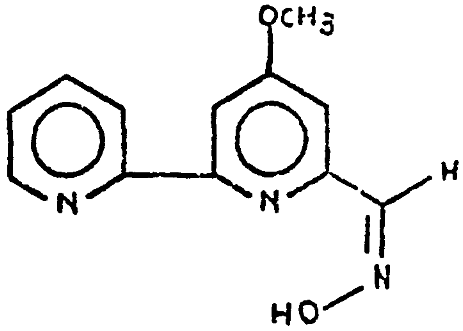

II

2. Verfahren zur Herstellung von Verbindungen der Formel I und II

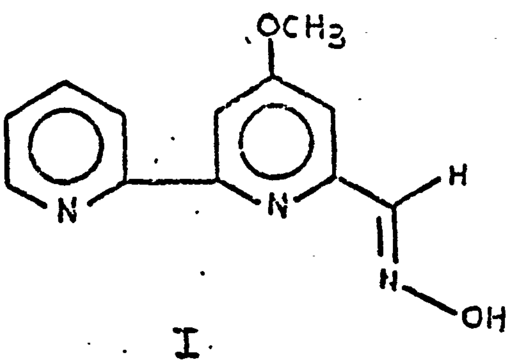

I

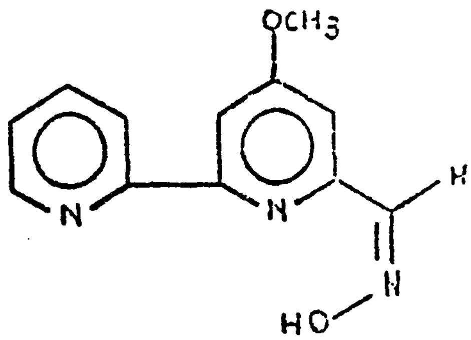

II

dadurch gekennzeichnet, daß man die Verbindung der Formel III

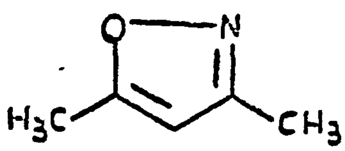

III

mit Lithiumdiisopropylamid und Pyridin-2-ethylcarboxylat zu der Verbindung der Formel IV

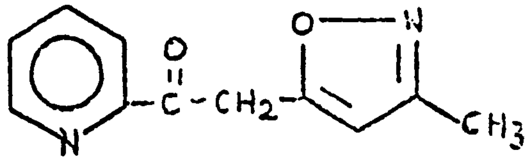

IV

umsetzt,
die erhaltene Verbindung in Gegenwart eines Platin-Katalysators hydriert zu der Verbindung der Formel V

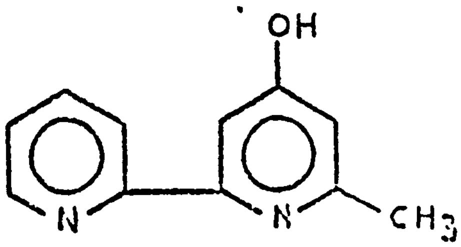

V

die erhaltene Verbindung mit Diazomethan umsetzt zu der Verbindung der Formel VI

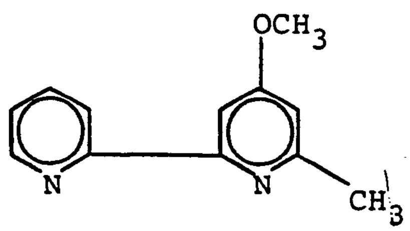

VI

die Verbindung der Formel VI umsetzt mit Lithiumdiisopropylamid und Butylnitrit zu einem Gemisch der Verbindungen der Formeln I und II, und das Gemisch in an sich bekannter Weise in die einzelnen Isomeren auftrennt.

3. Verfahren zur Herstellung von Verbindungen der Formeln I und II, dadurch gekennzeichnet, daß man die Verbindung der Formel

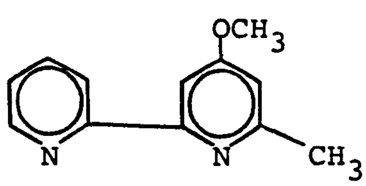

VI

mit Lithiumdiisopropylamid und anschließend mit Butylnitrit umsetzt zu einem Gemisch der Verbindungen der Formeln I und II und das Gemisch in an sich bekannter Weise auftrennt in die einzelnen Isomere.

4. Verbindung der Formel IV

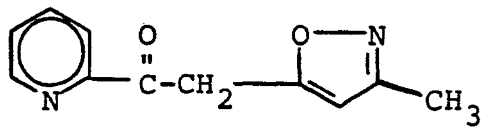

IV

5. Verbindung der Formel V

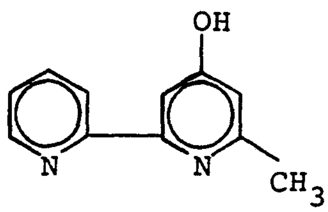

V

6. Verbindung der Formel VI

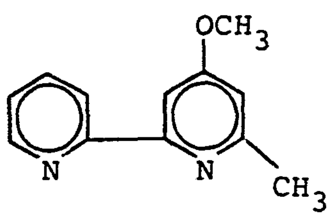

VI

7. Verwendung einer Verbindung der Formel I zur Behandlung von Krankheiten, die durch Coccidien oder Amöben hervorgerufen werden.

8. Verwendung einer Verbindung der Formel II zur Behandlung von Krankheiten, die durch Bakterien oder Amöben hervorgerufen werden.

0159640

Nummer der Anmeldung

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

EP 85104503.9

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A2/A3 - 0 045 277 (CIBA-GEIGY)<br>* Ansprüche 1,15,16 *<br>---- | 1,5,6 | C 07 D 213/68<br>C 07 D 413/06<br>A 61 K 31/44 |

RECHERCHIERTE SACHGEBIETE (Int. Cl.4)

C 07 D 213/00
C 07 D 413/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-6
Unvollständig recherchierte Patentansprüche: -
Nicht recherchierte Patentansprüche: 7,8
Grund für die Beschränkung der Recherche:

(Verfahren zur therapeutischen Behandlung des menschlichen oder tierischen Körpers (Artikel 52(4) EPÜ)

| Recherchenort | Abschlußdatum der Recherche | Prüfer |
|---|---|---|
| WIEN | 15-07-1985 | HOCHHAUSER |

KATEGORIE DER GENANNTEN DOKUMENTEN

- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1505.1 03.82